# EUROPEAN PATENT APPLICATION

(11) **EP 3 354 296 A1**
(43) Date of publication of application: **01.08.2018**
(21) Application number: 17153652.7
(22) Date of filing: 29.01.2017
(51) Int. Cl.: A61M 1/16

(54) **MEMBRANE OXYGENATION WITH REDUCED MEMBRANE SURFACE AREAS**

(71) Applicant: Köbrich, Rainer, 68809 Neulussheim (DE)
(72) Inventor: Köbrich, Rainer, 68809 Neulussheim (DE)
(74) Representative: Bast, Tim

(57) **Abstract**

A membrane oxygenation device comprising a gas-permeable membrane having a first surface and a second surface is provided. The first surface being exposed to blood and the second surface is exposed to an oxygen carrier liquid. In addition, a method for membrane oxygenation is provided.

The method comprises the steps of providing a gas-permeable membrane having a first surface and a second surface, exposing the first surface to blood, and simultaneously exposing the second surface to an oxygen carrier liquid.

## Description

### TECHNICAL FIELD

The present invention relates to the field of devices and methods for supporting blood oxygenation, more specifically to membrane oxygenation devices and methods for using them.

### BACKGROUND

In intensive care medicine, it is often necessary to support the blood oxygenation of a patient with external oxygenation devices during an acute respiratory stress syndrome, i.e. when the regular oxygenation through the patient's lungs has deteriorated below a critical level.

Extracorporeal membrane oxygenation (ECMO) devices have been used for longer term external blood oxygenation such as for periods of three to ten days. ECMO devices typically include a membrane for exchanging oxygen and carbon dioxide between a first volume through the patient's blood flows and a second volume into which a sweep gas is supplied. The membrane is impermeable for blood but permeable for oxygen and carbon dioxide. The effectiveness of the blood oxygenator depends on the surface of the membrane through which the gases are exchanged and the difference in partial pressures of oxygen and carbon dioxide between the blood and the sweep gas on the other side of the membrane.

The object of the present invention is to overcome the problems posed by the existing devices and methods for membrane oxygenation.

It is one aspect of the present invention to provide a device and method for membrane oxygenation that reduces the overall of the extra-corporeal contact surfaces for the blood flow in order to reduce the risk of blood coagulation and other damage to the blood.

It is another aspect of the present invention to provide a device and method for membrane oxygenation which increases the efficiency of transferring oxygen into the blood and removing carbon dioxide from the blood and thus reducing the required surface area of the gas-permeable membrane.

It is another aspect to provide a device and method for membrane oxygenation that allows reducing the extra-corporeal blood temperature loss without directly heating the blood in a heat exchanger.

It is another aspect to provide a device and method for membrane oxygenation that allows to control the blood temperature without directly exposing the blood to a heat exchanger.

### SUMMARY OF THE INVENTION

In one aspect the present invention is a membrane oxygenation device comprising a gas-permeable membrane having a first surface and a second surface. The first surface being exposed to blood and the second surface is exposed to an oxygen carrier liquid.

In another aspect, the present invention is a method for membrane oxygenation. The method comprises the steps of providing a gas-permeable membrane having a first surface and a second surface, exposing the first surface to blood, and simultaneously exposing the second surface to an oxygen carrier liquid.

These and other aspects of the invention are described in the claims and the below description and figures.

### BRIEF DESCRIPTION OF THE FIGURES

Fig. 1 is a schematic representation of a membrane oxygenation device.
Fig. 2 is a schematic representation of a membrane oxygenation device comprising a blood pump.
Fig. 3 is a schematic representation of a membrane oxygenation device comprising first and a second gar-permeable membrane.
Fig. 4 is a schematic representation of a membrane oxygenation device comprising a fluid pump as well as a heat exchanger for the oxygen carrier liquid.
Fig. 5 is a schematic representation of a membrane oxygenation device comprising a sweep gas reservoir.
Fig. 6 is a schematic representation of a membrane oxygenation device comprising control units for various elements.
Fig. 7 is a schematic representation of a membrane oxygenation device comprising a central control unit.

### DETAILED DESCRIPTION OF THE INVENTION

In one aspect, the present invention provides a membrane oxygenation device.

The term "**membrane oxygenation device**" as used herein refers to a device comprising a gas-permeable membrane having a first and a second surface across which oxygen and carbon dioxide may be exchanged. The membrane is impermeable for fluids such as blood so that fluids cannot permeate across the membrane from outside the first surface to outside of the second surface.

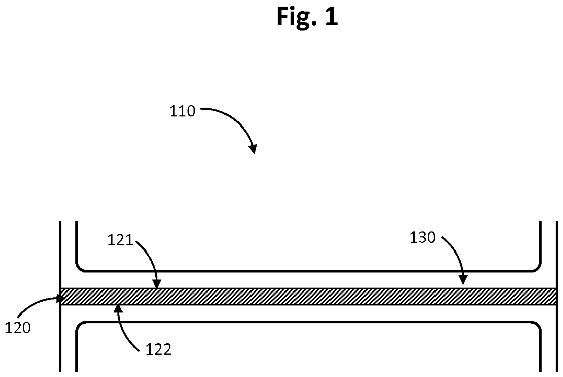

In Fig. 1, a schematic representation of a membrane oxygenation device 110 is depicted. The device comprises a gas permeable membrane 120 having a first surface 121 and a second surface 122.

Suitable gas-permeable membranes include without limitation the membranes used in state of the art oxygenators such as the Quadrox-i family of oxygenators commercially available from Maquet. Various spatial arrangements of the gas permeable membrane are suitable for use with the present invention such as planar, cylindrical, spherical, and combinations thereof. A preferred configuration is the configuration of a hollow fiber in which the first surface is facing inwardly and the second surface is facing outwardly. The blood is flowing through the internal volume of the hollow fiber.

The first surface 121 of the membrane 120 is exposed to the blood to be oxygenated. The second surface 122 of the membrane 120 is exposed to an oxygen carrier liquid.

The term "**oxygen carrier liquid**" means any liquid that is capable of supplying oxygen to blood and accepting carbon dioxide across the gas-permeable membrane by means of a difference in partial pressure. A suitable class of oxygen carrier liquids includes fluorocarbons, sometimes referred to as perfluorocarbons or PFCs. PFCs provide a high solubility for respiratory gases. PFCs are organofluorine compounds with the formula CₓF_{y}, i.e. they contain only carbon and fluorine atoms. Suitable PFCs for use with the present invention include without limitation the RhinoChill® collant commercially available from Benechill.

Using the oxygen carrier liquid in the membrane oxygenation device allows to reduce the effective size of the gas-permeable membrane or more specifically the membrane's surface because the effective rate of oxygenation using an oxygen carrier liquid has been found to be higher than when using a sweep gas. When blood is in contact with external surfaces, there exists a heightened risk of coagulation which increases when the surface area of the contact area is expanded. Reducing the size of the first surface of the gas permeable membrane will consequently reduce the risk of coagulation while the blood is oxygenated.

The blood to which the first surface of the gas-permeable membrane is exposed may be flowing inside a first volume 130 which is at least partially enclosed by the first surface of the membrane. The first volume 130 may comprise an inlet 131 through which blood may be supplied into the first volume 130. The blood may be supplied from the patient's body to the inlet 131. The blood may be venereal blood and arterial blood. The blood may be taken from a large vessel of the patient's body. The first volume may comprise an outlet 132 through which blood may leave the first volume. The outlet may be connected to a large vessel of the patient's body such as a large vein so that the blood flowing through the first volume may be returned to circulatory system of the patient's body.

Reducing the first and second surface areas of the gas-permeable membrane will also allow using an oxygenation element that has an overall smaller volume. This more effective element can then be placed closer to the patient's body which in turn reduces the length of the tubes required for connecting the inlet and the outlet of the first volume to the circulatory system of the patient's body. Using shorter tubes will further reduce the coagulation risk for the blood during the extra-corporeal oxygenation because the internal surface area of the tubing with which the blood comes into contact is reduced. Reducing the first and second surface areas further allows to use the oxygenation element in intracorporeal applications such as an implanted artificial lung.

The first volume of the membrane oxygenation device may comprise a blood pump to effect or assist the flow of blood from the patient's body to the inlet. The blood pump may be operationally positioned inside the first volume.

The term "**operably positioned inside**" a volume as used herein means that a blood pump is operably connected to a first part of the volume with its inlet and operably connected with its outlet to a second part of the volume such that in operation the blood pump is adapted to moving a liquid from the first part of the volume to the second part of the volume.

The blood pump may be positioned upstream or downstream of the gas-permeable membrane. Various types of suitable blood pumps are known in the art. A suitable type of blood pump is for example Rotaflow centrifugal pump commercially available from Maquet. Using a blood pump helps reducing the burden on the patient's circulatory system when pumping blood through the membrane oxygenation device. The pumping rate of the blood pump may be controlled such that the flow rate of the blood through membrane oxygenation device can be adjusted continuously in accordance with the medical needs. The control unit of the blood pump may be connected to a sensor measuring a physical condition of the patient's body such as the blood oxygenation level or the blood flow. The control unit may then be operable to adjust continuously an operational parameter of the blood pump such as the flow rate in dependence on the received sensor signal. The control unit may be operable to adjust the pump rate in dependence on a data signal received from a user interface device.

The blood pump may be positioned inside the first volume between the inlet and the portion of the first volume enclosed by the gas-permeable membrane. Placing the blood pump thus upstream of the gas-permeable membrane will reduce the burden on the patient's circulatory system to move the blood along the first membrane which may cause an increased friction due to its porous structure. The blood pump may directly control the flow rate of the blood along the first surface and thus to adjust immediately the oxygenation rate.

The outlet of the blood pump may be immediately connected to the portion of the first volume enclosed by the gas-permeable membrane.

The term "**immediately connected**" refers to a first volume and a second volume which are connected so that a liquid such as blood can flow from the first volume to the second volume without undergoing any further treatment steps and without being exposed to other treatment devices other than as necessary for facilitating the flow of the liquid.

The immediately connected blood pump may directly control the flow rate of the blood along the first surface and thus to adjust immediately the oxygenation rate.

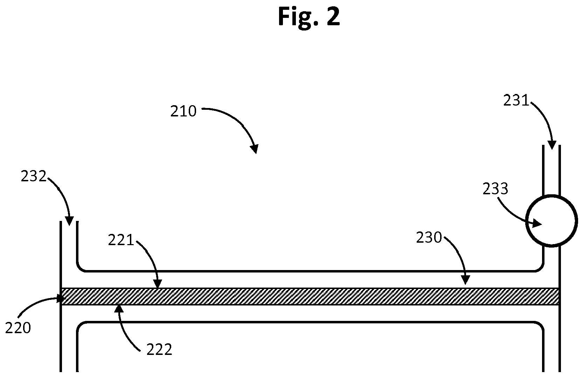

In Fig. 2, a membrane oxygenation device 210 comprising a blood is schematically depicted. The device comprises a blood pump 233 which is positioned inside the first volume 230 between the inlet 231 and the portion of the first volume 230 enclosed by the first surface 221 of the gas-permeable membrane 220.

The membrane oxygenation device may comprise a further inlet through which other fluids may be introduced into the blood flowing through the first volume. This further inlet may be positioned upstream of the blood pump. The additional fluid may be a medicament, such as heparin, dissolved in suitable fluid such a saline solution. Providing the further inlet offers the possibility to supply a medicament to the blood as well as to extract blood specimens for monitoring blood parameters. Supplying a medicament such as heparin to the blood prior to flowing across the first surface of the gas-permeable membrane allows to further reduce the risk of blood coagulation.

The portion of the first volume enclosed by the gas-permeable membrane is immediately connected to the outlet of the first volume. This immediate connection reduces the risk of adversely affecting the blood such as by creating further coagulation risks.

The oxygen carrier liquid may be flowing in a second volume having the form of a circuit whereby the second volume is partially enclosed by the gas-permeable membrane.

The second volume may further be partially enclosed from a second gas-permeable membrane. The second gas-permeable membrane may have a first surface and a second surface. The first surface of the second gas-permeable membrane may be exposed to the oxygen carrier liquid. The second surface of the gas-permeable membrane may be exposed to a sweep gas. Suitable gas-permeable membranes to be used at the second gas-permeable membrane include without limitation membranes used in state of the art oxygenators such as the Quadrox-i family of oxygenators commercially available from Maquet. Various spatial arrangements of the second gas-permeable membrane are suitable for use with the present invention such as planar, cylindrical, spherical, and combinations thereof. A preferred configuration is the configuration of a hollow fiber in which the first surface is facing inwardly and the second surface is facing outwardly. The oxygen carried liquid is flowing through the internal volume of the hollow fiber.

Sweep gases suitable for the present invention include without limitation medical oxygen as well as medical air or other suitable medical gases.

A pump for moving the oxygen carrier liquid may be operably positioned inside the second volume to move the oxygen carrier liquid along the circuit forming the second volume. A large variety of suitable pumps is available in the state of the art. A suitable type of pump is a centrifugal pump or a roller pump.

The pump rate of the pump for moving the oxygen carrier liquid may be controlled by a control unit. The control unit may be connected to a sensor measuring an external signal such as the oxygen saturation of the oxygen carrier liquid or the oxygen saturation of the blood either in the first circuit or inside the patient's circulatory system. The control unit may operable to the adjust the pump rate in dependence of a signal received from the sensor. The control unit may be adapted to adjust the pump rate in dependence on a data signal received from a user interface device. The control unit of the pump for moving the oxygen carrier liquid may be connected, for example with a bidirectional electrical connection, to the control unit of the pump for moving the blood in the first volume. Either control unit may be adapted for sending data signals to the other control units. Either control unit may be operable to adjust the rate of its pump in accordance with a data signal received from the other control unit.

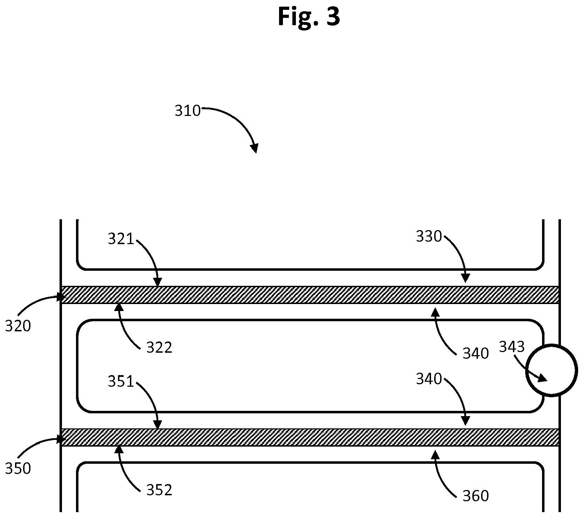

In Fig. 3, a membrane oxygenation device 310 is schematically depicted. The device comprises a second volume 340 in which the oxygen carrier liquid flows. The second volume 340 is partially enclosed by the first surface 351 of a second gas-permeable membrane 350. The second surface 352 of the second gas-permeable membrane 350 is exposed to a sweep gas flowing in a third volume 360. An optional pump 343 for moving the oxygen carrier liquid is operationally positioned inside the second volume 340.

A heat transfer device is positioned inside the second volume for controlling the temperature of the oxygen carrier liquid by either transferring heat to or extracting heat from the oxygen carrier liquid. Suitable heat transfer devices are well known in the art. A suitable type of heat transfer device is heater-cooler unit cardioplegia HU 35 commercially available from Maquet. The heat transfer device may comprise a control unit for controlling the amount of heat transferred to or extracted from the oxygen carrier liquid. The control unit may control the temperature of the oxygen carrier liquid directly through a heating element positioned inside the second volume. The control unit may control temperature of the oxygen carrier liquid indirectly by directly controlling the temperature of a secondary fluid such as water which is in turn transferring heat to or extracting heat from the oxygen carrier liquid through a fluid heat exchanger element in which the two liquids are placed to exchange heat without mixing with each other. The heat transfer device may comprise a control unit. The control unit may be connected to an external sensor which may send a data signal to the control unit. The control may be operable to adjust the temperature of the oxygen carrier liquid in dependence on the data signal received from the external sensor. The external sensor may be a sensor measuring the temperature of the blood flowing through the first volume or may be a sensor measuring the body temperature of the patient. The control sensor may be adapted to adjust the temperature of the oxygen carrier liquid in accordance with a data signal received from a user interface device. The control unit may be operable to continuously change the temperature of the oxygen carrier liquid from a first temperature to a second temperature at a predetermined constant or variable rate in order to externally control a change of body temperature of the patient.

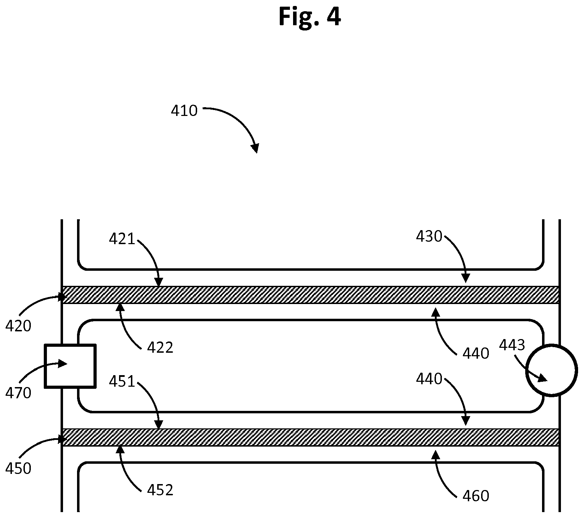

In Fig. 4, the membrane oxygenation device 410 is schematically represented. A heat transfer device 470 is positioned inside the second volume 440.

The membrane oxygenation device may comprise a third volume at least partially enclosed the second surface of the second gas-permeable membrane. The sweep gas may thus flow through the third volume coming into contact with the second surface of the second gas-permeable membrane. The third volume may have an inlet connected to a reservoir for sweep gas positioned upstream from the second surface of the second gas-permeable membrane. The sweep gas can thus flow from the reservoir into the third volume. The third volume may have an outlet for the sweep gas downstream second surface of the second gas-permeable membrane. An actuator for controlling the flow of sweep gas may be positioned inside the third volume or between the third volume the reservoir for sweep gas in order to control the flow rate of the sweep gas through the third volume. The actuator may form part of the reservoir and its outlet. The actuator may comprise a control unit for controlling the actuator. The control unit may be connected to a sensor for measuring an external parameter such as the oxygen saturation of the patient's blood, the blood in the first volume, or the oxygen carrier liquid in the second volume.

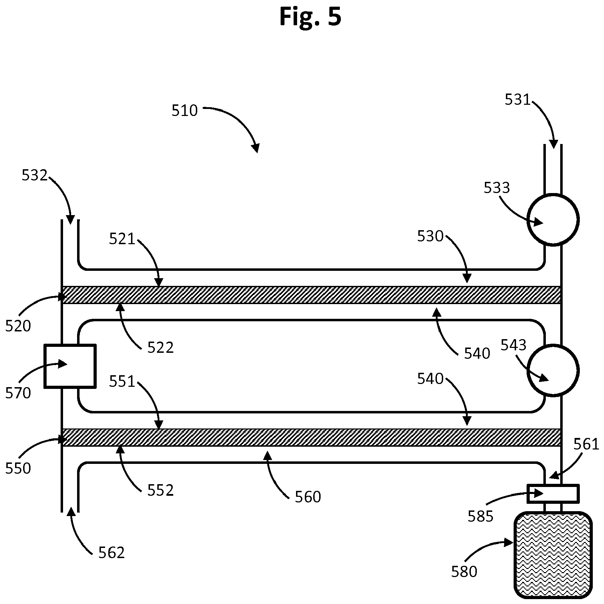

In Fig. 5, the membrane oxygenation device 510 is schematically represented. The second surface 552 of the second gas-permeable membrane 550 is exposed to sweep gas flowing through the third volume 560. The third volume 560 has an inlet 561 connected to a reservoir 580 for sweep gas positioned upstream from the second surface 552 of the second gas-permeable membrane 550. An actuator 585 for controlling the flow rate of the sweep gas is positioned inside the third volume 560 downstream of reservoir 580 and upstream of inlet 561.

Various units of the membrane oxygenation system such as the blood pump positioned, the pump for the oxygen carrier liquid, the heat transfer device, the actuator for controlling the sweep gas flow rate positioned inside the second volume may have control units. These control units may be separate and individually connected to the relevant unit.

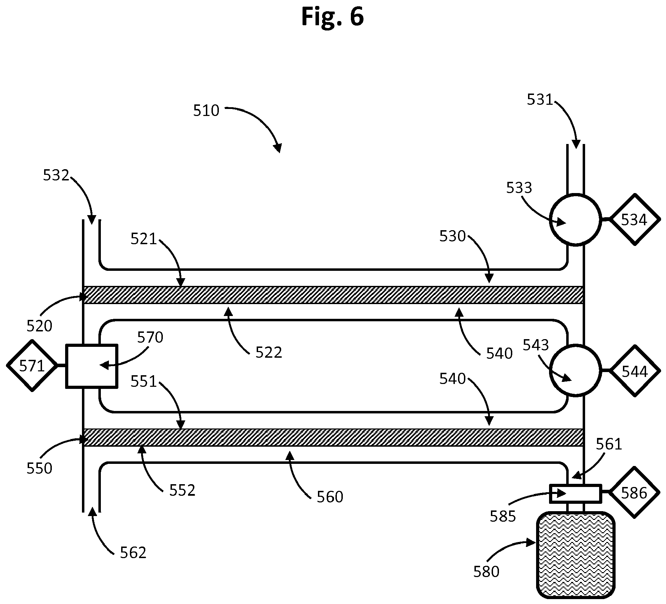

In Fig. 6, a schematic representation of a membrane oxygenation device 610 is depicted. The blood pump 633 is connected to control unit 634. The pump 643 for moving the oxygen carrier liquid is connected to control unit 644. The heat transfer device 670 is connected to control unit 671. The actuator 685 for controlling the flow rate of the sweep gas is connected to control unit 686.

The membrane oxygenation system may comprise a central control unit connected to all or any of the control units for the blood pump positioned in the first volume the pump for moving the oxygen carrier liquid, the control unit for the heat transfer device, or the control unit for actuator controlling the flow of sweep gas. The central control unit may be adapted to send data signals to and receive data signals from the other control units. Each of the other control units may be adapted to receive data signals and to send data signals to the central control unit. The central control unit and each of the other control units may change its controlling behaviour in accordance with the received data signals. The central control unit may comprise a user interface device. This user interface device may provide for user input or adjustment certain operational parameters. This user interface may be adapted to display certain operational data to the user such as the temperature or oxygen saturation of the blood in the first volume, the temperature or oxygen saturation of the oxygen carrier liquid, or the pump rate of either pump.

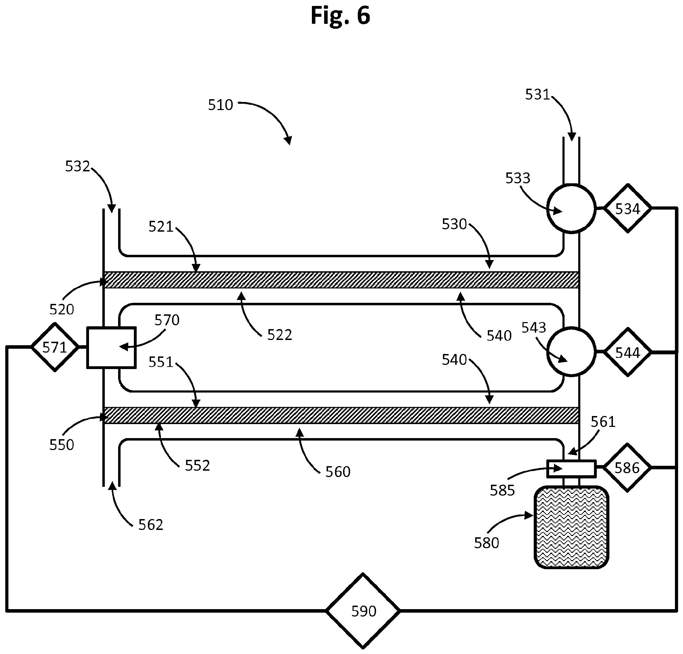

In Fig. 7, a schematic representation of the membrane oxygenation device 710 is depicted. All control units 734, 744, 771, and 786 are connected to central control unit 790.

In one aspect of the present invention, a method for membrane oxygenation is provided. The method comprises the steps of
- providing a gas-permeable membrane having a first surface and a second surface
- exposing the first surface to blood
- simultaneously exposing the second surface to an oxygen carrier liquid

The method for membrane oxygenation may further comprise the steps of
- providing a second gas-permeable membrane having a first and a second surface
- moving the oxygen carrier liquid in a circuit being partially enclosed the second surface of the first gas-permeable membrane and being partially enclosed by the first surface of the second gas-permeable membrane
- exposing the second surface of the second gas-permeable membrane to a sweep gas.

The method for membrane oxygenation may further comprise the step of controlling the temperature of the oxygen carrier liquid.

All of the methods for membrane oxygenation can be implemented using any one of the various membrane oxygenation devices described herein.

**LIST OF ELEMENTS**

| | |
|---|---|
| 10 | membrane oxygenation device |
| 20 | gas permeable membrane |
| 21 | first surface of the gas permeable membrane |
| 22 | second surface of the gas permeable membrane |
| 30 | first volume |
| 31 | inlet of first volume |
| 32 | outlet of first volume |
| 33 | blood pump |
| 34 | control unit for blood pump |
| 40 | second volume |
| 43 | pump for oxygen carrier liquid |
| 44 | control unit for pump for oxygen carrier liquid |
| 50 | second gas permeable membrane |
| 51 | first surface of the second gas permeable membrane |
| 52 | second surface of the second gas permeable membrane |
| 60 | third volume |
| 61 | inlet of third volume |
| 62 | outlet for third volume |
| 70 | heat transfer device |
| 71 | control unit for heat transfer device |
| 80 | reservoir for sweep gas |
| 85 | actuator for flow rate control |
| 90 | central control unit |

## Claims

1. Membrane oxygenation device (10) comprising a gas-permeable membrane (20) having a first surface (21) and a second surface (22), the first surface being exposed to blood
**characterized in that**
the second surface is exposed to an oxygen carrier liquid.

2. Membrane oxygenation device (10) according to Claim 1
wherein
the blood is flowing in a first volume (30) and the first volume is at least partially enclosed by the gas-permeable membrane (20).

3. Membrane oxygenation device (10) according to Claim 2
wherein
the first volume (30) has an inlet (31) and an outlet (32) and the membrane oxygenation device comprises a blood pump (33) operably positioned inside the first volume.

4. Membrane oxygenation device (10) according to Claim 3
wherein
the blood pump (33) is operably positioned inside the first volume (30) between the inlet and the portion enclosed by the gas-permeable membrane (20).

5. Membrane oxygenation device (10) according to any of Claim 3 or Claim 4
wherein
the inlet of the blood pump (33) is immediately connected to the inlet (31) of the first volume (30) and the outlet of the blood pump is immediately connected to the portion of the first volume enclosed by the gas-permeable membrane (20).

6. Membrane oxygenation device (10) according to any of Claim 2 through Claim 5
wherein
the portion of the first volume enclosed (30) by the gas-permeable membrane (20) is immediately connected to the outlet of the first volume (32).

7. Membrane oxygenation device (10) according to any of the preceding claims
wherein
the oxygen carrier liquid is flowing in a second volume (40) having the form of a circuit and the second volume is partially enclosed by the gas-permeable membrane (20).

8. Membrane oxygenation device (10) according to Claim 7
wherein
the second volume (40) is further partially enclosed from a second gas-permeable membrane (50) having a first surface (51) and a second surface (52), the first surface of the second gas-permeable membrane being exposed to the oxygen carrier liquid carrier and the second surface of the gas-permeable membrane being exposed to a sweep gas.

9. Membrane oxygenation device (10) according to any of Claim 7 or Claim 8
wherein
a pump for moving the oxygen carrier liquid (53) is operably positioned inside the second volume (40).

10. Membrane oxygenation device (10) according to any of Claim 7 through 9 further comprising a heat transfer device (70)
wherein
the heat transfer device is positioned inside the second volume (40) for controlling the temperature of the oxygen carrier liquid by either transferring heat to or extracting heat from the oxygen carrier liquid.

11. Membrane oxygenation device (10) according to any of Claim 8 through Claim 10
wherein
the membrane oxygenation device comprises a third volume (60) at least partially enclosed the second surface (52) of the second gas-permeable membrane (50),
the third volume has an inlet (61) connected to a reservoir (80) for sweep gas positioned upstream from the second surface of the second gas-permeable membrane,
the third volume has an outlet (62) for sweep gas downstream second surface of the second gas-permeable membrane, and
an actuator (85) for controlling the flow of sweep gas is positioned inside the third volume or between the third volume the reservoir for sweep gas.

12. A membrane oxygenation device (10) according to any of the preceding claims adapted for use as part of an artificial lung for intracorporeal application.

13. A method for membrane oxygenation comprising the steps of
- providing a gas-permeable membrane (10) having a first surface (11) and a second surface (12)
- exposing the first surface to blood
the method being **characterized by** further comprising the step of
- simultaneously exposing the second surface to an oxygen carrier liquid.

14. A method for membrane oxygenation according to Claim 13
wherein
the method further comprises the steps of
- providing a second gas-permeable membrane (50) having a first surface (51) and a second surface (52)
- moving the oxygen carrier liquid in a second volume (40) being partially enclosed the second surface (22) of the first gas-permeable membrane (20) and being partially enclosed by the first surface (51) of the second gas-permeable membrane (50)
- exposing the second surface of the second gas-permeable membrane to a sweep gas.

15. A method for membrane oxygenation according any of Claim 13 or Claim 14
wherein
the method further comprises the step of controlling the temperature of the oxygen carrier liquid.
